# EUROPEAN PATENT SPECIFICATION

(11) **EP 0 479 431 B1**
(45) Date of publication and mention of the grant of the patent: **08.12.1999**
(21) Application number: 91308054.5
(22) Date of filing: 03.09.1991
(51) Int. Cl.: C07D 405/06, C07D 205/085

(54) **Intermediates to 1-carbacephalosporins and process for preparation thereof**
Zwischenprodukte für 1-Carbacephalosporin und Verfahren zur Herstellung dieser
Produits intermédiaires pour 1-carbacéphalosporines et leur procédé de préparation

(30) Priority: 13.09.1990 US 582302
(43) Date of publication of application: 08.04.1992
(73) Proprietor: ELI LILLY AND COMPANY, Indianapolis, Indiana 46285 (US)
(72) Inventor: Aikins, James Abraham, Indianapolis, Indiana 46256 (US); Tao, Eddie Vi-Ping, Carmel, Indiana 46032 (US)
(74) Representative: Tapping, Kenneth George

(56) References cited:
- EP-A- 0 153 229
- EP-A- 0 252 744
- EP-A- 0 365 212
- US-A- 4 665 171
- CHEMICAL ABSTRACTS, vol. 106, no. 5, February 2, 1987, Columbus, Ohio, USA K. HIRAI et al. "An example of the beta-lactam ring formation and novel pyrrolinoazetidinone ring construction." page 519, column 1, abstract-no. 32 729t

## Description

### Field of the Invention

This invention generally relates to 1-carbacephalosporins and more particularly to intermediates to such compounds and processes for preparing those intermediates.

### Background of the Invention

Hashimoto et al. in U.S. Patent No. 4,335,211, incorporated herein by reference, disclose a class of 1-carbacephalosporins having desirable antibiotic and oral activity characteristics. These compounds are currently being evaluated for the treatment of various conditions such as the common upper- and lower-respiratory tract infections caused by the pathogen H. influenza. One such compound, 7-(R)-phenylglycinamido-3-chloro-1-azabicyclo(4,2,0]oct-2-en-8-on-2-carboxylic acid, known as loracarbef or LY163892, has shown activity against a broad spectrum of bacteria in laboratory tests. Loracarbef has proven to be a relatively stable compound which exhibits high blood levels and a relatively long half life.

The 1-carbacephalosporins thus far have not been obtained from natural sources, for example, as microbial metabolites. Accordingly, methods for the total synthesis of these promising compounds and for intermediates to these compounds are highly desirable, particularly methods which are adaptable to large scale manufacture, provide good yields, and reduce the cost of manufacture.

More particularly desired key intermediates to such compounds have the formula 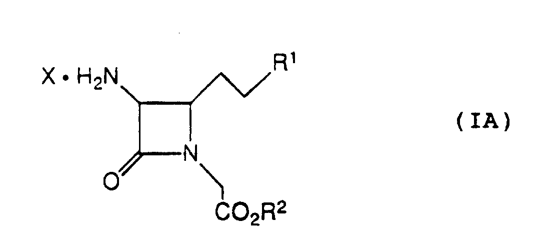 wherein R¹ is 2-furyl, phenyl, substituted phenyl, carboxy or protected carboxy, R² is C₁-C₆ alkyls, hydrogen, or a carboxy protecting group and X is an acid capable of forming acid addition salts. These intermediates represented by formula (IA) can then be further synthesized to form 1-carbacephalosporins, as disclosed generally in Evans et al., U.S. Patent No. 4,665,171, incorporated herein by reference.

A particular compound represented by formula (IA), a saturated methyl ester oxalate (IB), may be formed by a process as follows in Scheme 1.

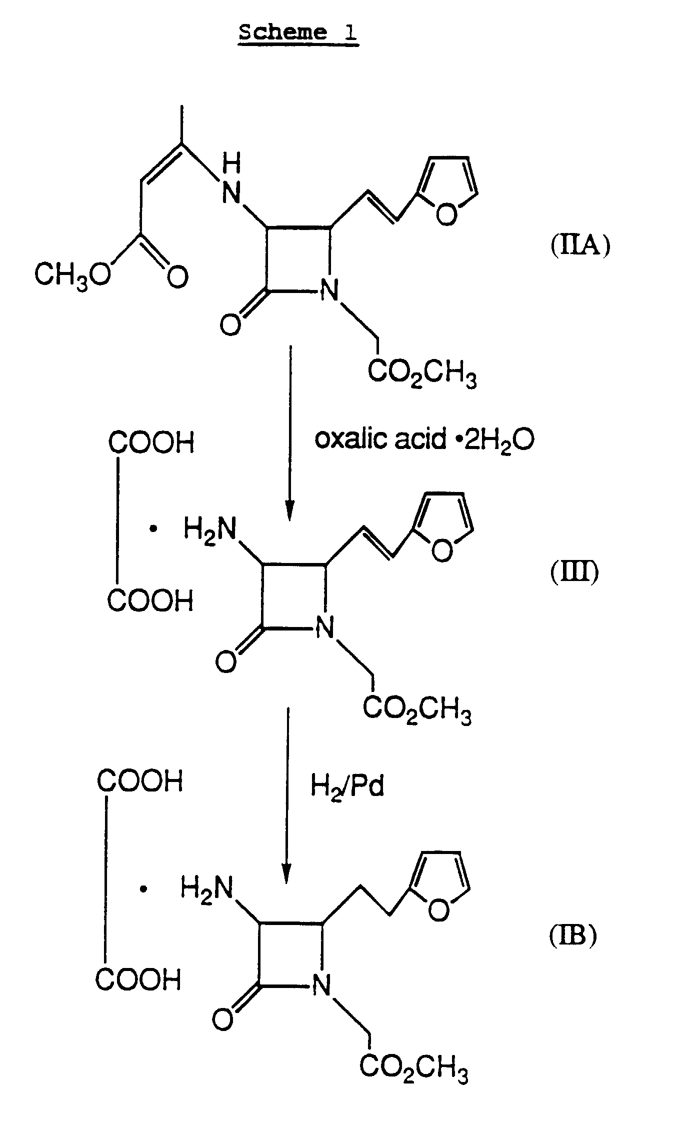

The process in Scheme 1 has approximately a 24 hour cycle time due, at least in part, to a relatively slow filtration of the compound as represented by formula (IB). Also, the intermediate represented by formula (III) is isolated before the ensuing hydrogenation step, and this also adds to the cycle time. Further, the process in Scheme 1 provides yields of the compound as represented by formula (IB) of approximately 20%. This relatively low yield may be due to ring cleavage and more particularly cleavage of the azetidinone ring resulting in an undesired compound having the formula

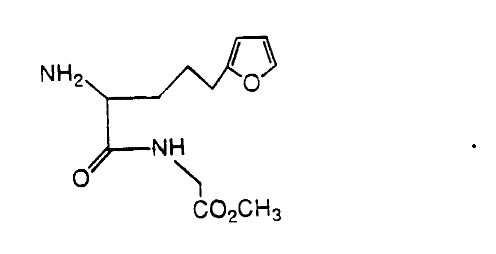

### Summary of the Invention

An object therefore is to provide a new and improved process for preparation of 1-carbacephalosporins and more particularly to a process for preparation of intermediates to 1-carbacephalosporins, and to novel intermediates.

The present invention provides a process for preparing a compound of the formula 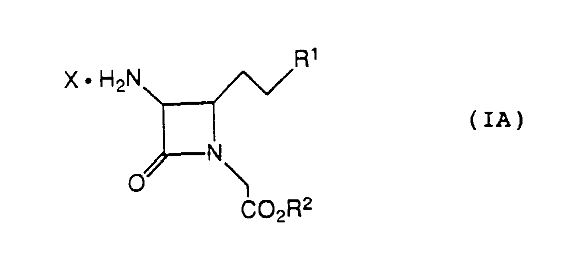 where R¹ is 2-furyl, phenyl, substituted phenyl, carboxy or protected carboxy, R² is a C₁-C₆ alkyl, hydrogen or a carboxy protecting group, and X is an acid capable of forming acid addition salts, and which includes the steps of hydrogenating a compound of the formula 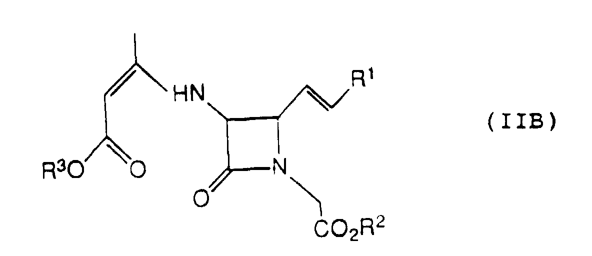 where R¹ and R² are defined as above and R³ is hydrogen, a C₁-C₆ alkyl or a carboxy protecting group, to form a compound having the formula 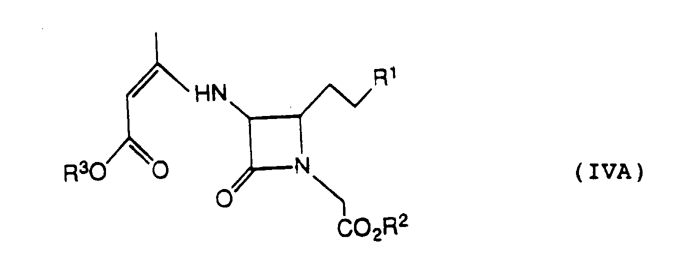 where R¹, R², and R³ are defined as above and thereafter reacting the compound of formula (IVA) with an acid as defined by X.

The present invention also provides novel intermediate compounds represented by formula (IVA).

While the entire scope of process variables taught herein are believed operable, the present process and intermediates do have exemplary aspects. Exemplary compounds of the above formulas are those in which R¹ is 2-furyl, R² is methyl, R³ is methyl, and X is oxalic acid.

Other objects and advantages will become apparent from the following description of the invention.

### Detailed Description of the Invention

The invention, together with its objects and advantages thereof, may be best understood by reference to the following description. The present examples and embodiments therefore are to be considered as illustrative.

The term "substituted phenyl" specifies a phenyl group substituted with one or more moieties chosen from the group consisting of halogen, hydroxy, protected hydroxy, cyano, nitro, C₁-C₆ alkyl, C₁-C₄ alkoxy, carboxy, protected carboxy, carboxymethyl, protected carboxymethyl, hydroxymethyl, protected hydroxymethyl, amino, protected amino, aminomethyl, protected aminomethyl, trifluoromethyl, or N-(methylsulfonylamino).

Examples of the term "substituted phenyl" include a mono- or di(halo)phenyl group such as 4-chlorophenyl, 2,6-dichlorophenyl, 2,5-dichlorophenyl, 3,4-dichlorophenyl, 3-chlorophenyl, 3-bromophenyl, 4-bromophenyl, 3,4-dibromophenyl, 3-chloro-4-fluorophenyl and 2-fluorophenyl ; a mono- or di-(hydroxy)phenyl group such as 4-hydroxyphenyl, 3-hydroxyphenyl, 2,4-dihyroxyphenyl, and the protected-hydroxy derivatives thereof ; a nitrophenyl group such as 3- or 4-nitrophenyl; a cyanophenyl group, for example 4-cyanophenyl; a mono- or di(lower alkyl)-phenyl group such as 4-methylphenyl, 2,4-dimethylphenyl, 2-methylphenyl, 4-(isopropyl)phenyl, 4-ethyl-phenyl, and 3-(n-propyl)phenyl ; a mono-or di(alkoxy)phenyl group, for example, 2,6-dimethoxyphenyl, 4-methoxyphenyl, 3-ethoxyphenyl, 4-(isopropoxy)-phenyl, 4-(t-butoxy)phenyl and 3-ethoxy-4-methoxyphenyl ; 3- or 4-trifluoromethylphenyl; a mono- or dicarboxyphenyl or (protected carboxy)phenyl group such as 4-carboxyphenyl or 2,4-di(protected carboxy)phenyl; a mono-or di(hydroxymethyl)phenyl or (protected hydroxymethyl)phenyl such as 3-(protected hydroxymethyl)phenyl or 3,4-di(hydroxymethyl)phenyl; a mono- or di(aminomethyl)phenyl or (protected aminomethyl)phenyl such as 2-(aminomethyl)phenyl or 2,4-(protected aminomethyl)-phenyl; or a mono- or di(N-(methylsulfonylamino))phenyl such as 3-(N-(methylsulfonylamino))phenyl. Also, the term "substituted phenyl" represents disubstituted phenyl groups wherein the substituents are different, for example, 3-methyl-4-hydroxyphenyl, 3-chloro-4-hydroxyphenyl, 2-methoxy-4-bromophenyl, 4-ethyl-2-hydroxyphenyl, 3-hydroxy-4-nitrophenyl and 2-hydroxy-4-chlorophenyl. Preferred substituted phenyl groups include the 2- and 3-trifluoromethylphenyl, the 4-hydroxyphenyl, the 2-aminomethylphenyl and the 3-(N-(methylsulfonylamino))phenyl groups.

The term "carboxy-protecting group" as used in the specification refers to one of the ester derivatives of the carboxylic acid group commonly employed to block or protect the carboxylic acid group while reactions are carried out on other functional groups on the compound. Examples of such carboxylic acid protecting groups include 4-nitrobenzyl, 4-methylbenzyl, 3,4-dimethoxybenzyl, 2,4-dimethoxybenzyl, 2,4,6-trimethoxybenzyl, 2,4,6-trimethylbenzyl, pentamethylbenzyl, 3,4-methylenedioxybenzyl, benzhydryl, 4,4'-dimethoxybenzhydryl, 2,2',4,4'-tetramethoxybenzhydryl, t-butyl, t-amyl, trityl, 4-methoxytrityl, 4,4'-dimethoxytrityl, 4,4',4"-trimethoxytrityl, 2-phenylprop-2-yl, trimethylsilyl, t-butyldimethylsilyl, phenacyl, 2,2,2-trichloroethyl, β-(di(n-butyl)methylsilyl)ethyl, p-toluenesulfonylethyl, 4-nitrobenzylsulfonylethyl, allyl, cinnamyl and 1-(trimethylsilylmethyl)prop-1-en-3-yl. The species of carboxy-protecting group employed is not critical so long as the derivatized carboxylic acid is stable to the condition of subsequent reaction(s) on other positions of the azetidinone ring and can be removed at the appropriate point without disrupting the remainder of the molecule. (Such harsh removal conditions are also to be avoided when removing amino-protecting groups and hydroxy-protecting groups.) A preferred carboxylic acid protecting group is the allyl group. Similar carboxy-protecting groups used in the cephalosporin, penicillin and peptide arts can also be used to protect a carboxy group substituents of the azetidinone. Further examples of these groups are found in E. Haslam, "Protective Groups in Organic Chemistry", J.G.W. McOmie, Ed., Plenum Press, New York, N.Y., 1973, Chapter 5, and T.W. Greene, "Protective Groups in Organic Synthesis", John Wiley and Sons, New York, N.Y., 1981, Chapter 5. The related terms "protected carboxy" and "protected carboxymethyl" denote that a carboxy group is substituted with one of the above carboxy-protecting groups.

The term "hydroxy-protecting group" refers to readily cleavable groups bonded to hydroxyl groups, such as the tetrahydropyranyl, 2-methoxyprop-2-yl, 1-ethoxyeth-1-yl, methoxymethyl, β-methoxyethoxymethyl, methylthiomethyl, t-butyl, t-amyl, trityl, 4-methoxytrityl, 4,4'-dimethoxytrityl, 4,4',4"-trimethoxytrityl, benzyl, allyl, trimethylsilyl, (t-butyl)dimethylsilyl and 2,2,2-trichloroethoxycarbonyl groups.

Further examples of hydroxy-protecting groups are described by C.B. Reese and E. Haslam, "Protective Groups in Organic Chemistry", J.G.W. McOmie, Ed., Plenum Press, New York, N.Y., 1973, Chapters 3 and 4, respectively, and T.W.Greene, "Protective Groups in Organic Synthesis", John Wiley and Sons, New York, N.Y., 1981, Chapters 2 and 3. Some preferred hydroxy-protecting groups are the trityl group and the tetrahydropyranyl group. The related terms "protected hydroxy" and "protected hydroxymethyl" denote that a hydroxy group is bonded to one of the above hydroxy-protecting groups.

The term "amino-protecting group" refers to substituents of the amino group commonly employed to block or protect the amino functionality while reacting other functional groups on the compound. Examples of such amino-protecting groups include the formyl group, the trityl group, the phthalimido group, the trichloroacetyl group, the chloroacetyl, bromoacetyl and iodoacetyl groups, urethane-type blocking groups such as benzyloxycarbonyl, 4-phenylbenzyloxycarbonyl, 2-methylbenzyloxycarbonyl, 4-methoxybenzyloxycarbonyl, 4-fluorobenzyloxycarbonyl, 4-chlorobenzyloxycarbonyl, 3-chlorobenzyloxycarbonyl, 2-chlorobenzyloxycarbonyl, 2,4-dichlorobenzyloxycarbonyl, 4-bromobenzyloxycarbonyl, 3-bromobenzyloxycarbonyl, 4-nitrobenzyloxycarbonyl, 4-cyanobenzyloxycarbonyl, 2-(4-xenyl)isopropoxycarbonyl, 1,1-diphenyleth-1-yloxycarbonyl,1,1-diphenylprop-1-yloxycarbonyl, 2-phenylprop-2-yloxycarbonyl, 2-(p-toluyl)prop-2yloxycarbonyl, cyclopentanyloxycarbonyl, l-methylcyclopentanyloxycarbonyl, cyclohexanyloxycarbonyl, l-methylcyclohexanyloxycarbonyl, 2-methylcyclohexanyloxycarbonyl, 2-(4-toluylsulfonyl)ethoxycarbonyl, 2-(methylsulfonyl)ethoxycarbonyl, 2-(triphenylphosphino)ethoxycarbonyl, 9-fluorenylmethoxycarbonyl ("FMOC"), 2-(trimethylsilyl)ethoxycarbonyl, allyloxycarbonyl, 1-(trimethylsilylmethyl)prop-l-enyloxycarbonyl, 5benzisoxalylmethoxycarbonyl, 4-acetoxybenzyloxycarbonyl, 2,2,2-trichloroethoxycarbonyl, 2-ethynyl-2-propoxycarbonyl, cyclopropylmethoxycarbonyl, 4-(decyloxy)benzyloxycarbonyl, isobornyloxycarbonyl and 1-piperidyloxycarbonyl ; the benzoylmethylsulfonyl group, the 2-(nitro)phenylsulfenyl group and, the diphenylphosphine oxide group. Preferred amino-protecting groups are the allyloxycarbonyl, the t-butoxycarbonyl, and the trityl groups. Similar amino-protecting groups used in the cephalosporin, penicillin and peptide art are also embraced by the above terms. Further examples of groups referred to by the above terms are described by J. W. Barton, "Protective Groups In Organic Chemistry", J. G. W. McOmie, Ed., Plenum Press, New York, N.Y., 1973, Chapter 2, and T. W. Greene, "Protective Groups in Organic Synthesis", John Wiley and Sons, New York, N.Y., 1981, Chapter 7. The related terms "protected amino" and "protected aminomethyl" denote that an amino group is substituted with an amino-protecting group discussed above.

The present invention provides a process for the preparation of intermediates to 1-carbacephalosporin following Scheme 2. 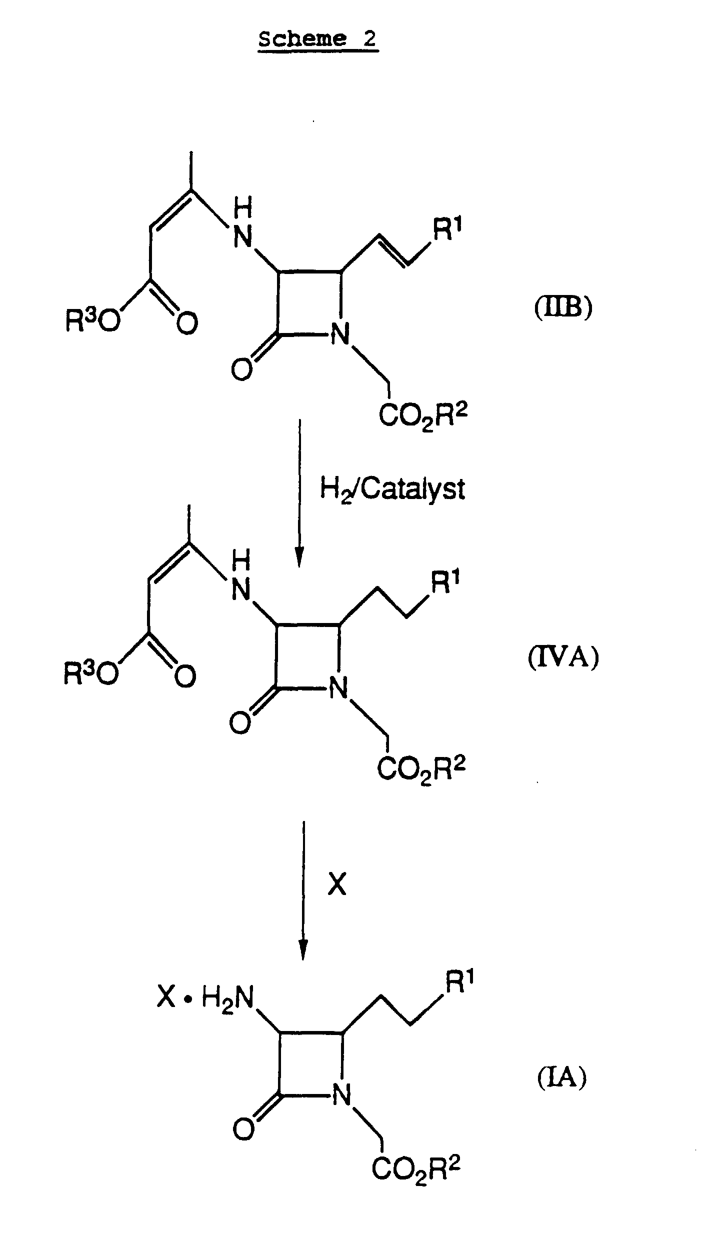 wherein R¹ is 2-furyl, phenyl, substituted phenyl, carboxy or protected carboxy, R² is a C₁-C₆ alkyl, hydrogen or a carboxy protecting group, R³ is hydrogen, a C₁-C₆ alkyl or a carboxy protecting group, and X is an acid capable of forming acid addition salts. More preferably, R² is a C₁-C₆ alkyl or a carboxy protecting group and R³ is a C₁-C₆ alkyl or a carboxy protecting group.

The starting compounds as represented by formula (IIB), enamino β-lactams, are readily prepared by prior art processes. For example, Bose, U.S. Patent No. 4,260,743, incorporated herein by reference, teaches synthesis of compounds generally of the configuration of formula (IIB), by activating the carboxyl group of a vinylamino salt of the formula 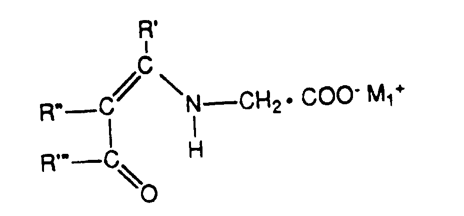 wherein R' represents lower alkyl, aryl or aryl(lower-alkyl), R" represents hydrogen or lower alkyl or R' and R" together with the carbon atom to which they are attached represent lower cycloalkyl and R"' represents a lower alkyl or a group -OR, wherein R represents lower alkyl, and wherein M₁+ is the cation of a base, with an appropriate activating agent and reacting the activated compound in the presence of a tertiary base with an imino compound (Schiff's base) of the formula 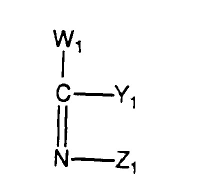 wherein W₁, Y₁, and Z₁ are hydrogen or selected organic radicals, using, however, the following Schiff's base 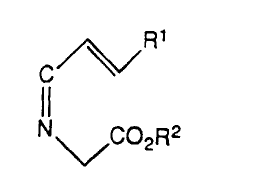 wherein R¹ and R² are defined as previously.

The hydrogenation in the present invention takes place in the presence of a standard hydrogenation catalyst. Hydrogenation catalysts include nickel, platinum, rhodium, ruthenium, copper chromite, iridium, osmium, palladium, and combinations thereof. An exemplary catalyst is a supported palladium, e.g., 5% or 10% palladium on carbon, barium carbonate, or other suitable support. The reduction generally is carried out at atmospheric conditions or at somewhat elevated pressures, and at substantially room temperature.

The intermediate represented by formula (IVA) is then reacted with an acid capable of forming acid addition salts such as the desired compound of formula (IA). Acids employed to form the addition salts must be relatively strong, and preferably have pKa values of about 2 or less. Such acids include inorganic acids such as hydrochloric acid, hydrobromic acid, hydroiodic acid, sulfuric acid and phosphoric acid, and organic acids such as maleic, p-toluenesulfonic, methanesulfonic acid, oxalic acid, p-bromophenylsulfonic acid, and halogenated acetic acid. Preferably, the acid is oxalic acid or oxalic acid dihydrate.

Polar organic cosolvents may be used as precipitants to remove starting material and for isolating and cleanup. One such precipitant is acetonitrile.

The process of the invention is a "one-pot", two-step process and avoids the need to isolate an intermediate, which affords savings in time, equipment and man hours in manufacturing the key intermediate represented by formula (IA). Further, the filtration of the compound represented by formula (IA) is carried out relatively quickly, which aids in reducing overall cycle time relative to the prior art process. Also, the yield provided by the process of the invention is substantially higher than that of the process in Scheme 1 herein described.

The following examples further illustrate the specific aspects of the present invention.

### Experimental Section

The starting material for the experiments illustrating the invention has the formula 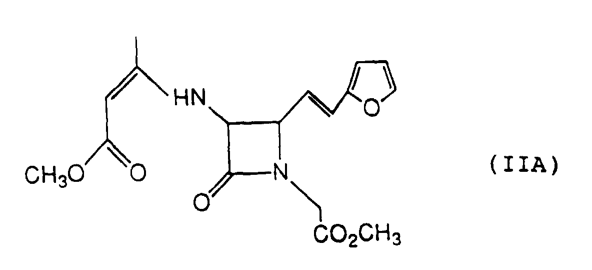 while the intermediate formed after hydrogenation has the formula 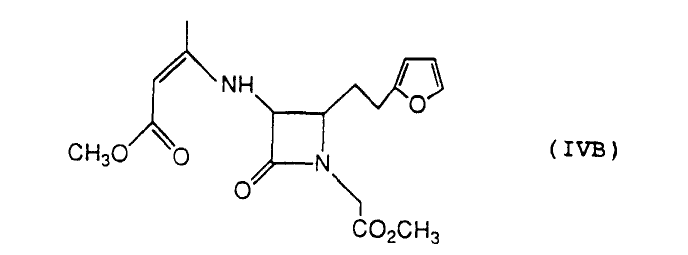 and the key intermediate formed has the formula

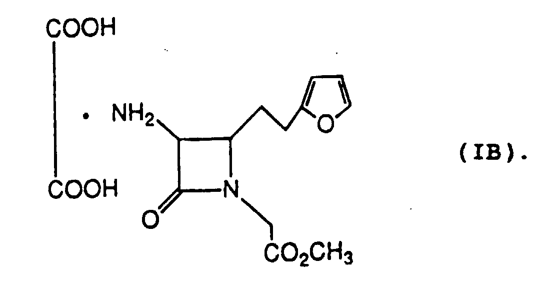

### Example 1

An 89.09 gram sample of the compound represented by formula (IIA) was added to 0.8 gram of 5% palladium on carbon. The mixture was then subjected to a hydrogen atmosphere at 1·4 bar (20 psi) and at room temperature for three hours and twenty minutes. HPLC indicated very little starting material after the hydrogenation. The palladium catalyst was removed by filtration, and the mixture was placed in a rotary evaporator at a temperature of about 25-40°C to concentrate the mixture to an oil. To the mixture, 350 ml of acetonitrile, 16.6 grams of oxalic acid, and 1 ml of water was added and the mixture was stirred for two and one-half hours. The mixture was chilled to 0-5°C, then filtered. The cake was washed with 500 ml of acetonitrile. The precipitate was dried in a vacuum at a temperature of approximately 40°C.

The dried precipitate had a mass of 19.42 grams which represented a percent weight yield of 61.6% of the theoretical mass (31.5 grams). The purity indicated by HPLC of the above precipitate was 95.7% of the compound of formula (IB), resulting in a corrected yield of 59%.

### Example 2

To 0.10 liters of the compound of formula (IIA), 1.64 grams of 5% palladium on carbon was added . The mixture was hydrogenated at 1·4 bar (20 psi) and at room temperature for three hours. At that time, HPLC indicated that 29% of the starting material was still left. Therefore, the mixture was refrigerated overnight after which hydrogenation was continued for another four and one-half hours at which time HPLC indicated no starting material present. The palladium catalyst was then removed by filtration and the mixture washed with 75 ml of methylene chloride. The mixture was concentrated to an oil by heating in a rotary evaporator at a temperature of about 25-40°C. To the mixture, 650 ml of acetonitrile and 21.4 grams of oxalic acid dihydrate were added. The mixture was stirred for two hours and then chilled to 0-5°C. The precipitate was then filtered and washed with 800 ml of acetonitrile and the precipitate was dried in vacuo. The mass of the resulting precipitate (compound of formula (IB)) was 38.13 grams and had a percent weight yield of 65.7% of theory, (58.06 grams).

It is noteworthy that the compound of the following formula 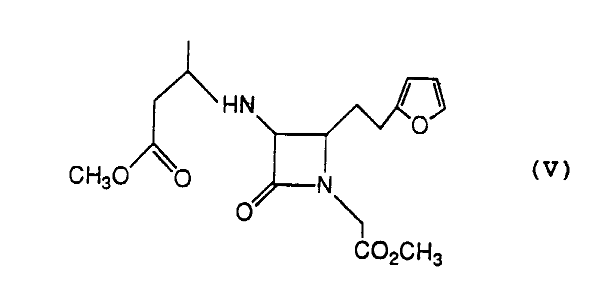 was not produced in the hydrogenation step in either of the examples. This was a concern as production of a compound represented by formula (V) would be detrimental to further synthesis according to the invention because one would be left with a 3-alkylamino substituent which could not be readily converted to a useful 3-amino intermediate.

## Claims (Claims for the following Contracting State(s): AT, BE, CH, LI, DE, DK, FR, GB, GR, IT, LU, NL, SE)

1. A process for the preparation of compounds having the formula 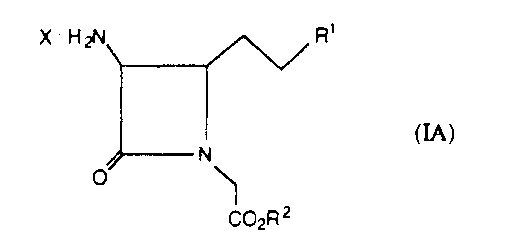 wherein R¹ is 2-furyl, phenyl, substituted phenyl, which comprises a phenyl group substituted with one or more moieties chosen from halogen, hydroxy, protected hydroxy, cyano, nitro, C₁-C₆ alkyl, C₁-C₄ alkoxy, carboxy, protected carboxy, carboxymethyl, protected carboxymethyl, hydroxymethyl, protected hydroxymethyl, amino, protected amino, aminomethyl, protected aminomethyl, trifluoromethyl or N-(methyl-sulfonylamino), carboxy or protected carboxy, R₂ is a C₁-C₆ alkyl, hydrogen, or a carboxy protecting group and X is an acid capable of forming acid addition salts, comprising the steps of:
hydrogenating a compound of the formula 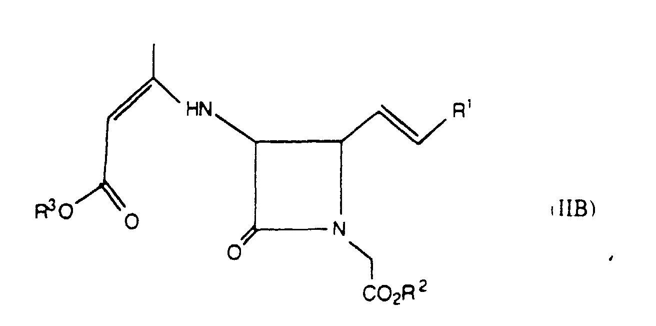 wherein R¹ and R² are defined as above and R³ is hydrogen, a C₁-C₆ alkyl, or a carboxy protecting group, in the presence of a hydrogenation catalyst, to form a compound having the formula 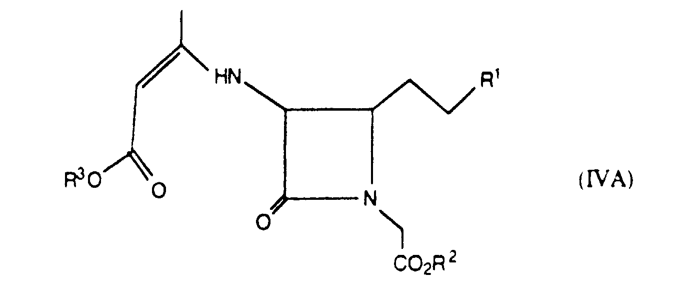 wherein R¹, R², and R³ are defined as above and thereafter, without isolating the intermediate compound, reacting the compound of formula (IVA) with an acid as defined by X.

2. A compound represented by the formula 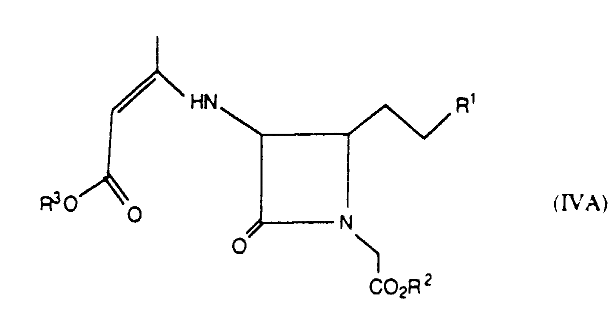 wherein R¹ is 2-furyl, phenyl, substituted phenyl, which comprises a phenyl group substituted with one or more moieties chosen from halogen, hydroxy, protected hydroxy, cyano, nitro, C₁-C₆ alkyl, C₁-C₄ alkoxy, carboxy, protected carboxy, carboxymethyl, protected carboxymethyl, hydroxymethyl, protected hydroxymethyl, amino, protected amino, aminomethyl, protected aminomethyl, trifluoromethyl or N-(methyl-sulfonylamino), carboxy or protected carboxy, R² is a C₁-C₆ alkyl, hydrogen or a carboxy protecting group, and R³ is hydrogen, a C₁-C₆ alkyl or a carboxy protecting group.

3. The process or compound as recited in claim 1 or 2 wherein R¹ is 1-furyl, R² is methlyl, and R³ is methyl.

4. The process as recited in claims 1 wherein X is oxalic acid or oxalic acid dihydrate.

5. The process as recited in claims 1 or 4 wherein said hydrogenation catalyst is palladium.

## Claims (Claims for the following Contracting State(s): ES)

1. A process for the preparation of compounds having the formula 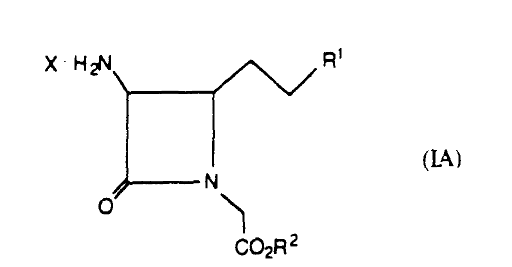 wherein R¹ is 2-furyl, phenyl, substituted phenyl, which comprises a phenyl group substituted with one or more moieties chosen from halogen, hydroxy, protected hydroxy, cyano, nitro, C₁-C₆ alkyl, C₁-C₄ alkoxy, carboxy, protected carboxy, carboxymethyl, protected carboxymethyl, hydroxymethyl, protected hydroxymethyl, amino, protected amino, aminomethyl, protected aminomethyl, trifluoromethyl or N-(methyl-sulfonylamino), carboxy or protected carboxy, R₂ is a C₁-C₆ alkyl, hydrogen, or a carboxy protecting group and X is an acid capable of forming acid addition salts, comprising the steps of:
hydrogenating a compound of the formula 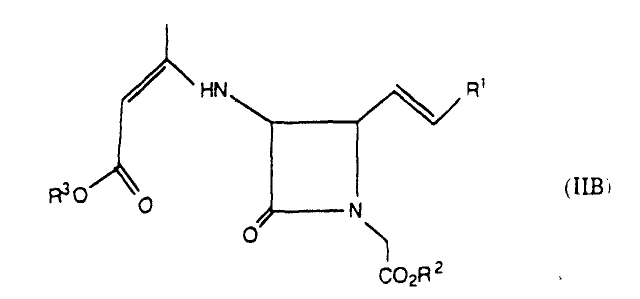 wherein R¹ and R² are defined as above and R³ is hydrogen, a C₁-C₆ alkyl, or a carboxy protecting group, in the presence of a hydrogenation catalyst, to form a compound having the formula 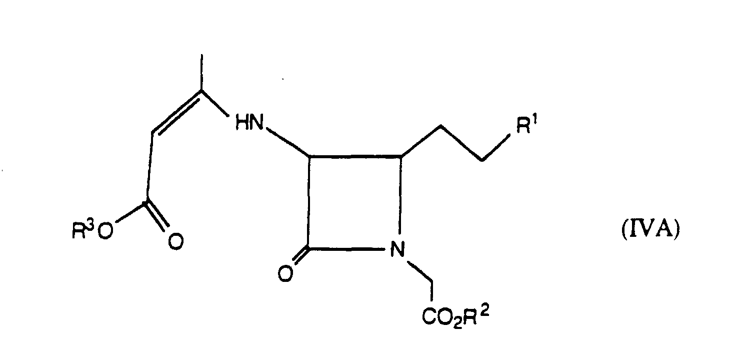 wherein R¹, R², and R³ are defined as above and thereafter, without isolating the intermediate compound, reacting the compound of formula (IVA) with an acid as defined by X.

2. The process of claim 1 wherein R¹ is 1-furyl, R² is methyl, and R³ is methyl.

3. The process of claims 1 wherein X is oxalic acid or oxalic acid dihydrate.

4. The process of claims 1 or 3 wherein said hydrogenation catalyst is palladium.

## Patentansprüche (Patentansprüche für folgende(n) Vertragsstaat(en): AT, BE, CH, LI, DE, DK, FR, GB, GR, IT, LU, NL, SE)

1. Verfahren zur Herstellung von Verbindungen der Formel 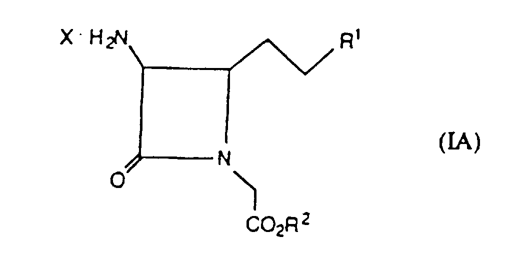 worin R¹ steht für 2-Furyl, Phenyl, substituiertes Phenyl, das eine Phenylgruppe umfaßt, die mit einem oder mehreren Resten substituiert ist, ausgewählt aus Halogen, Hydroxy, geschütztes Hydroxy, Cyano, Nitro, C₁-C₆ Alkyl, C₁-C₄ Alkoxy, Carboxy, geschütztes Carboxy, Carboxymethyl, geschütztes Carboxymethyl, Hydroxymethyl, geschütztes Hydroxymethyl, Amino, geschütztes Amino, Aminomethyl, geschütztes Aminomethyl, Trifluormethyl oder N-(Methylsulfonylamino), für Carboxy oder geschütztes Carboxy, R² für ein C₁-C₆ Alkyl, Wasserstoff oder eine Carboxyschutzgruppe steht und X für eine Säure steht, die zur Bildung von Säureadditionssalzen fähig ist, gekennzeichnet durch die folgenden Schritte:
Hydrierung einer Verbindung der Formel 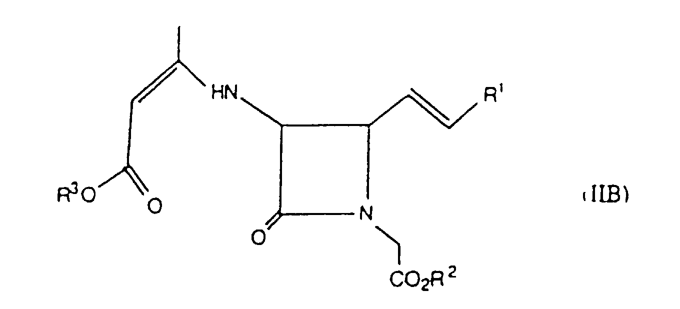 worin R¹ und R² wie oben definiert sind und R³ für Wasserstoff, ein C₁-C₆ Alkyl oder eine Carboxyschutzgruppe steht, in Gegenwart eines Hydrierungskatalysators unter Bildung einer Verbindung der folgenden Formel 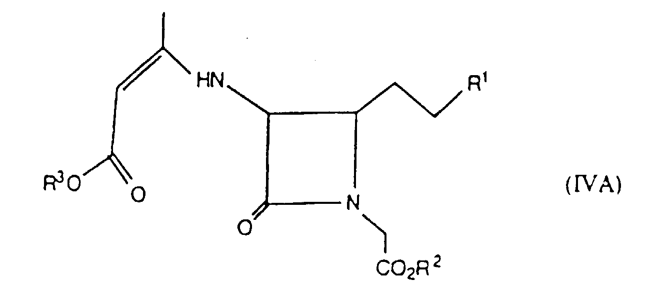 worin R¹, R² und R³ wie oben definiert sind und danach ohne Isolierung der Zwischenproduktverbindung, Umsetzung einer Verbindung der Formel (IVA) mit einer durch X definierten Säure.

2. Verbindung der Formel 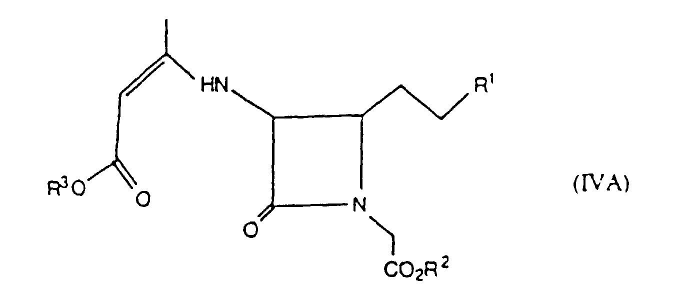 worin R¹ steht für 2-Furyl, Phenyl, substituiertes Phenyl, das eine Phenylgruppe umfaßt, die mit einem oder mehreren Resten substituiert ist, ausgewählt aus Halogen, Hydroxy, geschütztes Hydroxy, Cyano, Nitro, C₁-C₆ Alkyl, C₁-C₄ Alkoxy, Carboxy, geschütztes Carboxy, Carboxymethyl, geschütztes Carboxymethyl, Hydroxymethyl, geschütztes Hydroxymethyl, Amino, geschütztes Amino, Aminomethyl, geschütztes Aminomethyl, Trifluormethyl oder N-(Methylsulfonylamino), für Carboxy oder geschütztes Carboxy, R² für ein C₁-C₆ Alkyl, Wasserstoff oder eine Carboxyschutzgruppe steht und R³ für Wasserstoff, ein C₁-C₆ Alkyl oder eine Carboxyschutzgruppe steht.

3. Verfahren oder Verbindung nach Anspruch 1 oder 2, worin R¹ für 1-Furyl, R² für Methyl und R³ für Methyl steht.

4. Verfahren nach Anspruch 1, worin X für Oxalsäure oder Oxalsäuredihydrat steht.

5. Verfahren nach einem der Ansprüche 1 oder 4, worin der Hydrierungskatalysator Palladium ist.

## Patentansprüche (Patentansprüche für folgende(n) Vertragsstaat(en): ES)

1. Verfahren zur Herstellung von Verbindungen der Formel 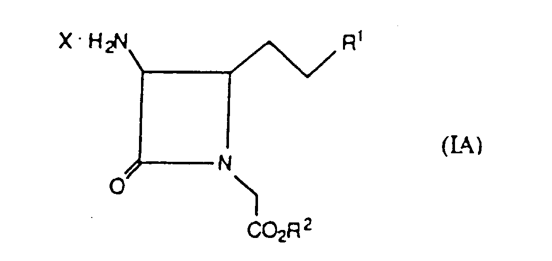 worin R¹ steht für 2-Furyl, Phenyl, substituiertes Phenyl, das eine Phenylgruppe umfaßt, die mit einem oder mehreren Resten substituiert ist, ausgewählt aus Halogen, Hydroxy, geschütztes Hydroxy, Cyano, Nitro, C₁-C₆ Alkyl, C₁-C₄ Alkoxy, Carboxy, geschütztes Carboxy, Carboxymethyl, geschütztes Carboxymethyl, Hydroxymethyl, geschütztes Hydroxymethyl, Amino, geschütztes Amino, Aminomethyl, geschütztes Aminomethyl, Trifluormethyl oder N-(Methylsulfonylamino), für Carboxy oder geschütztes Carboxy, R² für ein C₁-C₆ Alkyl, Wasserstoff oder eine Carboxyschutzgruppe steht und X für eine Säure steht, die zur Bildung von Säureadditionssalzen fähig ist, gekennzeichnet durch die folgenden Schritte:
Hydrierung einer Verbindung der Formel 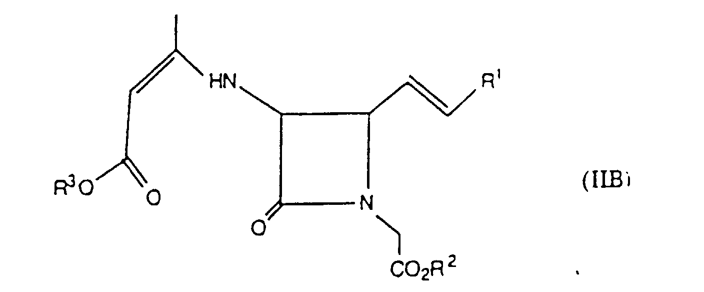 worin R¹ und R² wie oben definiert sind und R³ für Wasserstoff, ein C₁-C₆ Alkyl oder eine Carboxyschutzgruppe steht, in Gegenwart eines Hydrierungskatalysators unter Bildung einer Verbindung der folgenden Formel 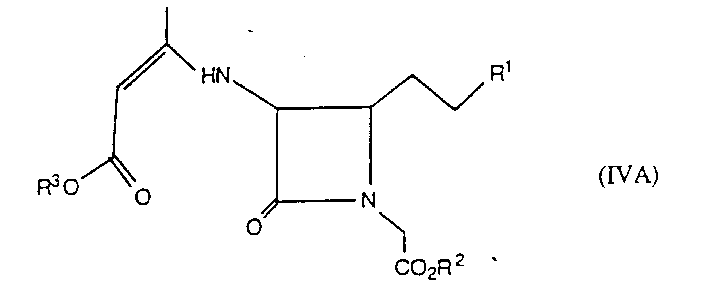 worin R¹, R² und R³ wie oben definiert sind und danach ohne Isolierung der Zwischenproduktverbindung, Umsetzung einer Verbindung der Formel (IVA) mit einer durch X definierten Säure.

2. Verfahren nach Anspruch 1 oder 2, worin R¹ für 1-Furyl, R² für Methyl und R³ für Methyl steht.

3. Verfahren nach Anspruch 1, worin X für Oxalsäure oder Oxalsäuredihydrat steht.

4. Verfahren nach einem der Ansprüche 1 oder 3, worin der Hydrierungskatalysator Palladium ist.

## Revendications (Revendications pour l'(les) Etat(s) contractant(s) suivant(s): AT, BE, CH, LI, DE, DK, FR, GB, GR, IT, LU, NL, SE)

1. Procédé pour la préparation de composés répondant à la formule 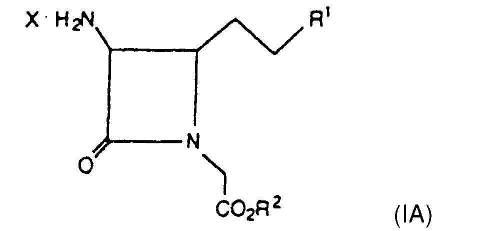 dans laquelle R¹ représente un groupe 2-furyle, un groupe phényle, un groupe phényle substitué qui comprend un groupe phényle substitué par une ou plusieurs fractions choisies parmi un atome d'halogène, un groupe hydroxyle, un groupe hydroxyle protégé, un groupe cyano, un groupe nitro, un groupe alkyle en C₁-C₆, un groupe alcoxy en C₁-C₄, un groupe carboxyle, un groupe carboxyle protégé, un groupe carboxyméthyle, un groupe carboxyméthyle protégé, un groupe hydroxyméthyle, un groupe hydroxyméthyle protégé, un groupe amino, un groupe amino protégé, un groupe aminométhyle, un groupe aminométhyle protégé, un groupe trifluorométhyle ou un groupe N-(méthylsulfonylamino), un groupe carboxyle ou un groupe carboxyle protégé, R₂ représente un groupe alkyle en C₁-C₆, un atome d'hydrogène ou un groupe protecteur du groupe carboxyle et X représente un acide capable de former des sels d'addition acide, comprenant les étapes de :
hydrogénation d'un composé répondant à la formule 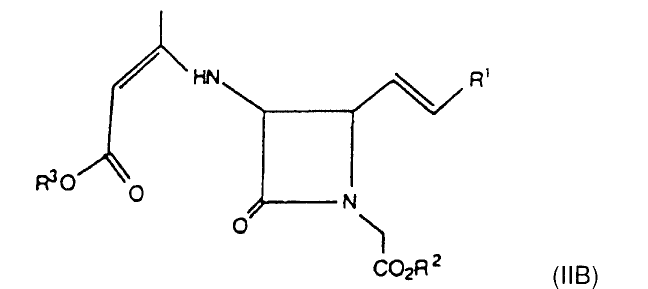 dans laquelle R¹ et R² sont tels que définis ci-dessus et R³ représente un atome d'hydrogène, un groupe alkyle en C₁-C₆ ou un groupe protecteur du groupe carboxyle, en présence d'un catalyseur d'hydrogénation de façon à former un composé répondant à la formule 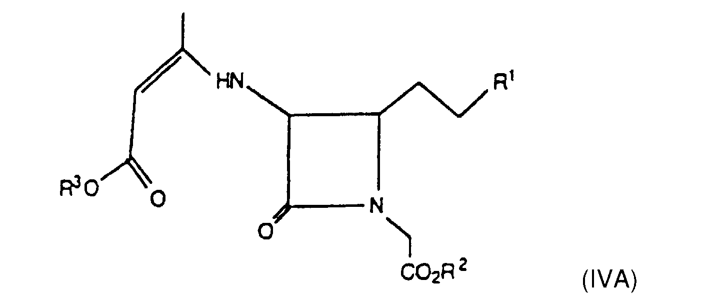 dans laquelle R¹, R² et R³ sont tels que définis ci-dessus et ensuite, sans isolement du composé intermédiaire, réaction du composé répondant à la formule (IVA) avec un acide tel que défini par X.

2. Composé représenté par la formule 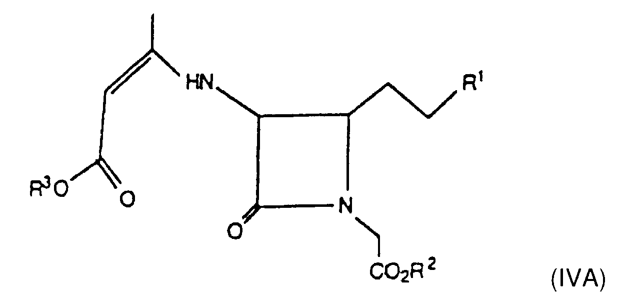 dans laquelle R¹ représente un groupe 2-furyle, un groupe phényle, un groupe phényle substitué qui comprend un groupe phényle substitué par une ou plusieurs fractions choisies parmi un atome d'halogène, un groupe hydroxyle, un groupe hydroxyle protégé, un groupe cyano, un groupe nitro, un groupe alkyle en C₁-C₆, un groupe alcoxy en C₁-C₄, un groupe carboxyle, un groupe carboxyle protégé, un groupe carboxyméthyle, un groupe carboxyméthyle protégé, un groupe hydroxyméthyle, un groupe hydroxyméthyle protégé, un groupe amino, un groupe amino protégé, un groupe aminométhyle, un groupe aminométhyle protégé, un groupe trifluorométhyle ou un groupe N-(méthylsulfonylamino), un groupe carboxyle ou un groupe carboxyle protégé, R² représente un groupe alkyle en C₁-C₆, un atome d'hydrogène ou un groupe protecteur du groupe carboxyle, R³ représente un atome d'hydrogène, un groupe alkyle en C₁-C₆ ou un groupe protecteur du groupe carboxyle.

3. Procédé ou composé selon la revendication 1 ou 2, dans lequel R¹ représente un groupe 1-furyle, R² représente un groupe méthyle et R³ représente un groupe méthyle.

4. Procédé selon la revendication 1, dans lequel X représente l'acide oxalique ou l'acide oxalique dihydrate.

5. Procédé selon la revendication 1 ou 4, dans lequel ledit catalyseur d'hydrogénation est le palladium.

## Revendications (Revendications pour l'(les) Etat(s) contractant(s) suivant(s): ES)

1. Procédé pour la préparation de composés répondant à la formule 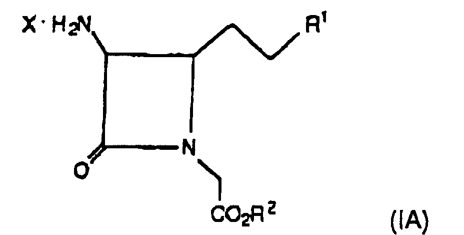 dans laquelle R¹ représente un groupe 2-furyle, un groupe phényle, un groupe phényle substitué qui comprend un groupe phényle substitué par une ou plusieurs fractions choisies parmi un atome d'halogène, un groupe hydroxyle, un groupe hydroxyle protégé, un groupe cyano, un groupe nitro, un groupe alkyle en C₁-C₆, un groupe alcoxy en C₁-C₄, un groupe carboxyle, un groupe carboxyle protégé, un groupe carboxyméthyle, un groupe carboxyméthyle protégé, un groupe hydroxyméthyle, un groupe hydroxyméthyle protégé, un groupe amino, un groupe amino protégé, un groupe aminométhyle, un groupe aminométhyle protégé, un groupe trifluorométhyle ou un groupe N-(méthylsulfonylamino), un groupe carboxyle ou un groupe carboxyle protégé, R₂ représente un groupe alkyle en C₁-C₆, un atome d'hydrogène ou un groupe protecteur du groupe carboxyle et X représente un acide capable de former des sels d'addition acide, comprenant les étapes de :
hydrogénation d'un composé répondant à la formule 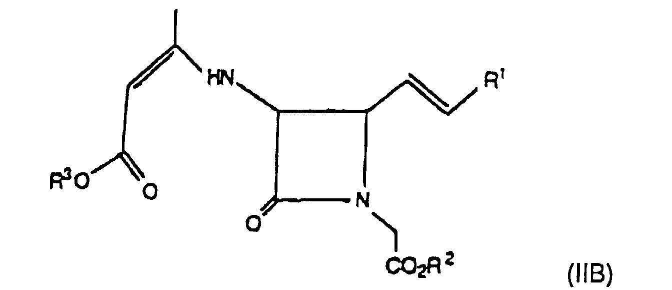 dans laquelle R¹ et R² sont tels que définis ci-dessus et R³ représente un atome d'hydrogène, un groupe alkyle en C₁-C₆ ou un groupe protecteur du groupe carboxyle, en présence d'un catalyseur d'hydrogénation de façon à former un composé répondant à la formule 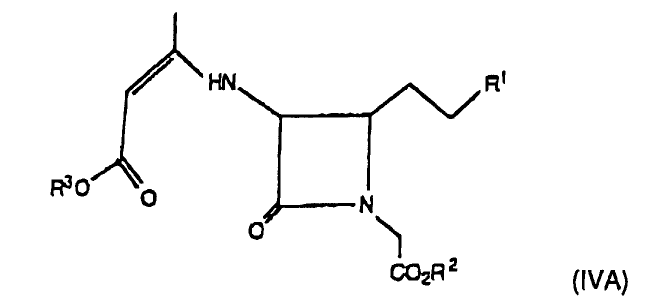 dans laquelle R¹, R² et R³ sont tels que définis ci-dessus et ensuite, sans isolement du composé intermédiaire, réaction du composé répondant à la formule (IVA) avec un acide tel que défini par X.

2. Procédé selon la revendication 1, dans lequel R¹ représente un groupe 1-furyle, R² représente un groupe méthyle et R³ représente un groupe méthyle.

3. Procédé selon la revendication 1, dans lequel X représente l'acide oxalique ou l'acide oxalique dihydrate.

4. Procédé selon la revendication 1 ou 3, dans lequel ledit catalyseur d'hydrogénation est le palladium.
